# EUROPEAN PATENT SPECIFICATION

(11) **EP 1 818 064 B1**
(45) Date of publication and mention of the grant of the patent: **13.11.2013**
(21) Application number: 07001730.6
(22) Date of filing: 26.01.2007
(51) Int. Cl.: A61K 8/41, A61K 8/67, A61Q 19/00, A61Q 19/10, A61K 47/12, A61K 9/00, A61K 47/18

(54) **Methods of treating skin to enhance therapeutic treatment thereof**
Verfahren zur Behandlung der Haut zur Verstärkung der therapeutischen Effekte
Méthode de traitement de la peau en améliorant l'activité thérapeutique

(30) Priority: 02.02.2006 US 764410 P; 02.02.2006 US 764503 P; 22.12.2006 US 644206; 26.12.2006 US 645405
(43) Date of publication of application: 15.08.2007
(73) Proprietor: OMP, Inc., Long Beach, CA 90802 (US)
(72) Inventor: Ramirez, José E., Trumbull, CT 06611 (US); Faryniarz, Joseph, Middlebury, CT 06762 (US)
(74) Representative: Thoma, Michael

(56) References cited:
- WO-A-2006/060515
- US-B1- 6 197 813
- MONTI D ET AL: "Comparison of the effect of ultrasound and of chemical enhancers on transdermal permeation of caffeine and morphine through hairless mouse skin in vitro" INTERNATIONAL JOURNAL OF PHARMACEUTICS 20011028 NL, vol. 229, no. 1-2, 28 October 2001 (2001-10-28), pages 131-137, XP002523797 ISSN: 0378-5173
- SINHA V R ET AL: "Permeation enhancers for transdermal drug delivery" DRUG DEVELOPMENT AND INDUSTRIAL PHARMACY 2000 US, vol. 26, no. 11, 2000, pages 1131-1140, XP002523798 ISSN: 0363-9045

## Description

### BACKGROUND

### Technical Field

The present disclosure relates to methods for attaining excellent percutaneous absorption of topically applied actives. More specifically, this disclosure relates to pre-treatment of an area of a user's skin with one or more cationic constituents to enhance penetration of an active. The scope of protection is defined by the claims.

### Background of Related Art

Document Monti D et al.: "Comparison of the effect of ultrasound and of chemical enhancers on transdermal permeation of caffeine and morphine through hairless mouse skin in vitro", INTERNATIONAL JOURNAL OF PHARMACEUTICS 20011028 NL, vol. 229, no. 1-2, 28 October 2001 (2001-10-28), pages 131-137, XP002523797, ISSN: 0378-5173 describes the effect of pretreatment of skin with different chemical enhancers including benzalkonium chloride on the permeation of caffeine and morphine through hairless mouse skin, wherein it is mentioned that such pretreatment significantly increases the permeation of morphine. Furthermore, document WO 2006/060515 is directed to sequential application of a cleanser, toner and a composition containing an active ingredient to the skin, wherein the said cleanser includes cationic ingredients like cocamidopropyl betaine.

The topical application of active ingredients to improve skin characteristics rarely, if ever attains the maximum benefit possible. The condition of the skin to which the active is applied may diminish the effectiveness of the active. For example, the availability of positively charged molecules on the surface of skin can affect abilities of certain drugs to be absorbed into the skin once applied and diminish the effectiveness of the active ingredient. Low availability of positively charged molecules may lead to low absorption and significantly decrease the effectiveness of active ingredient resulting in the need for frequent and/or prolonged application of the active to obtain a desired beneficial effect.

The prior art is problematic in that it does not demonstrate improving percutaneous absorption of an active ingredient by pre-treating skin with a cationic constituent for the immediate penetration of drug or active ingredient. Accordingly, there remains room for improvement in skin treatment regimens that improve percutaneous absorption of an active ingredient in skin in need of treatment.

### SUMMARY

The present invention provides for the use of a salt of an alkyldimethylbenzylamine for the manufacture of a composition for improving the percutaneous absorption of ascorbic acid topically applied to the skin of a user, as defined by claim 1. Preferred embodiments of the invention are laid down in the dependent claims.

Pre-treating skin by topically applying one or more cationic constituents such as cationic salt or cation containing solution, and then topically applying an active ingredient increases the percutaneous absorption of active ingredient in skin.

Methods in accordance with embodiments of the present disclosure involve specific steps in a skin therapy or treatment system that can be better than the individual steps performed alone. The skin treatment system in accordance with this disclosure includes the sequential steps of pre-treating the skin with one or more cationic constituents such as cationic salt or cation containing solution, to render the area more receptive to the application of a drug or active ingredients thereto; and applying one or more drugs or other active ingredients to the pre-treated area.

The present disclosure further relates to a method which includes the step of applying one or more pre-treatment agents to an area of skin in order to actively enhance penetration of the active ingredients, and/or enhances long term penetration of one or more drugs or active ingredients. In embodiments, the present disclosure relates to applying a pre-treatment cationic constituent. The present methods may result in an increase in the presence of an active ingredient in two levels of the skin (i.e., the epidermis, and dermis), compared to application of the active ingredient alone.

### DESCRIPTION OF THE PREFERRED EMBODIMENTS

Treatment regimens in accordance with this disclosure include the sequential steps of: pre-treating the surface of skin with a cationic constituent; and applying an active ingredient to the skin, wherein the percutaneous absorption of the active ingredient is increased compared to application of the active ingredient to untreated skin.

The first step of the present method is pre-treatment. The pre-treatment step of the treatment regimen of the present disclosure is designed for rendering the area of skin to be treated more receptive to the application of the drug(s) or active ingredients thereto. It has been found that applying a cationic constituent such as salt or cationic solution actively promotes penetration of drugs and active ingredients applied to the skin surface. Cleaning and/or toning in combination with the cationic constituent may further promote drug penetration.

Materials suitable for use as pre-treatment agents include preparatory compositions pre-selected to apply positive charge to skin in need of treatment, and/or clean or tone skin. Non-limiting examples of preparatory compositions are listed below and include cationic constituents such as cationic salt compositions and cationic solutions, cleanser compositions, and/or toner compositions.

### PREPARATORY COMPOSITIONS

In accordance with the present disclosure, one or more cationic constituents can be applied in amounts that provide the benefit to the skin of the user, such as in an amount sufficient to coat skin surface with positively charged molecules. As used herein, cationic constituent refers to any ingredient with one or more positively charged moieties that apply positive charge to skin. Non-limiting examples of cationic constituents include one or more cationic molecules such as cationic salts, positively charged amino acids, quaternary ammonium salts, cationic polymers, and combinations of these cationic constituents.

In embodiments, cationic salts suitable for use as a pre-treatment in accordance with the present disclosure include cationic salts that are not substantially toxic at the dosage administered to achieve the desired effect and do not independently possess significant pharmacological activity. Non-limiting examples of suitable cationic salts include cationic salts formed at any acidic (e.g., carboxyl) group; multivalent cationic salts, including cations of the alkaline earth metals (Group IIA), transition metals (Groups IIB, IVB, VB, VIIB, VIIB, VIIIB, IB, IIB, IIA, IVA) and non-metal elements (Groups IVA, VA) for use alone or combined together, or in combination with other cationic constituents described herein. In embodiments, cationic salts include positively charged salts of carboxymethylcellulose (CMC). In embodiments, cationic salts include the alkali metal salts (such as, for example, sodium and potassium), alkaline earth metal salts (such as, for example, magnesium and calcium), and organic salts.

Pharmaceutically acceptable cationic salts suitable for use in accordance with the present disclosure include any salts formed by neutralization of the free carboxylic acid group of pharmacologically active compounds. The neutralization occurs by contacting the carboxylic acid containing compounds with a base of a pharmaceutically acceptable metal, ammonia and amine. Non-limiting examples of such metals are sodium, potassium, calcium and magnesium. Non-limiting examples of such amines are N-methylglucamine and ethanolamine.

In embodiments, cationic salts include those organic primary, secondary and tertiary amines, as for example, trialkylamines, including triethylamine, procaine, dibenzylamine, 1-ethenamine, N,N'-dibenzylethylenediamine, dihydroabiethylamine, N-(lower)alkylpiperidine, and any other suitable amine. Alkyldimethylbenzylamine salts are particularly useful cationic salts.

In embodiments, suitable cationic constituents contain amino acids having a positive charge. Non-limiting examples of suitable amino acids which can be positively charged include alanine, arginine, asparagine, aspartic acid, cysteine, glutamine, glutamic acid; glycine, histidine, isoleucine, leucine, lysine, methionine, phenylalanine, proline, serine, threonine, tryptophan, tyrosine, and valine.

In embodiments, basic amino acids are suitable for use herein. Basic amino acids are those amino acids where in a neutral solution, the R group of the amino acid can gain a proton and become positively charged. Non-limiting examples of suitable basic amino acids include lysine, arginine, and histidine.

Non-limiting examples of suitable quaternary compounds include: benzalkonium chlorides and/or substituted benzalkonium chlorides, dialkyl quaternary, N-(3-chloroallyl) hexaminium chlorides, benzethonium chloride, methylbenzethonium chloride, and cetylpyridinium chloride. In embodiments, benzalkonium chloride such as, for example, a 5% solution can be applied to the skin before application of an active solution such as a vitamin C formulation.

Non-limiting examples of cationic polymer suitable for use in accordance with the present disclosure include cationic polysaccharides, cationic copolymers of saccharides and synthetic cationic monomers, and synthetic polymers.

Non-limiting examples of cationic polysaccharides includes polymers based on a 5 or 6 carbon sugars and derivatives, which have been made cationic by adding cationic moieties onto the polysaccharide backbone. Such cationic polysaccharides can be composed of one type of sugar or of more than one type, i.e. copolymers. The monomers may be in straight chain or branched chain geometric arrangements. Additional suitable cationic polysaccharide polymers include: cationic celluloses and hydroxyethyl celluloses, cationic starches and hydroxyalkyl starches, cationic polymers based on arabinose monomers, cationic polymers derived from xylose polymers, cationic polymers derived from fucose polymers, cationic polymers derived from fructose polymers, cationic polymers based on acid-containing sugars such as galacturonic acid and glucuronic acid, cationic polymers based on amine sugars such as galactosamine and glucosamine, cationic polymers based on 5 and 6 membered ring polyalcohols, cationic polymers based on galactose monomers which occur in plant gums and mucilages, cationic polymers based on mannose monomers, and cationic polymers based on galactomannan copolymer known as guar gum.

The cationic copolymers of saccharides and synthetic cationic monomers in accordance with the present disclosure include those containing the following saccharides: glucose, galactose, mannose, arabinose, xylose, fucose, fructose, glucosamine, galactosamine, glucuronic acid, galacturonic acid, and 5 or 6 membered rinse polyalcohols. Also included are hydroxymethyl, hydroxyethyl and hydroxypropyl derivatives of the above saccharides. When saccharides are bonded to each other in the copolymers, they may be bonded via any of several arrangements, such as 1, 4-.alpha.; 1, 4-.beta.; 1, 3-.alpha.; 1, 3-.beta. and 1, 6 linkages. The synthetic cationic monomers for use in these copolymers can include dimethyldiallylammonium chloride, dimethylaminoethylmethyacrylate, diethyldiallyl ammonium chloride, N,N-diallyl,N-N-dialklyl ammonium halides, and the like. In embodiments, cationic polymer is Polyquaternium -5, -6, -7, and combinations of these polyquaternium compositions.

Non-limiting examples of copolymers of saccharides and synthetic cationic monomers include those composed of cellulose derivatives (e.g. hydroxyethyl cellulose) and N,N-diallyl,N-N-dialkyl ammonium chloride.

Non-limiting examples of cationic synthetic polymers include: cationic polyalkylene imines, cationic ethoxypolyalkylene imines, cationic poly[N-[3-(dimethylammonio)propyl]-N'[3-(ethyleneoxyethylene dimethylammonio)propyl]urea dichloride], and polymers having a quaternary ammonium or substituted ammonium ion. In embodiments, cationic polymeric skin conditioning agents are those cationic polysaccharides of the cationic guar gum with molecular weights of 1,000 to 3,000,000. Such polymers have a polysaccharide backbone comprised of galactomannan units and a degree of cationic substitution ranging from about 0.04 per anydroglucose unit to about 0.80 per anydroglucose unit with the substituent cationic group being the adduct of 2,3-epoxypropyl-trimethyl ammonium chloride to the natural polysaccharide backbone.

Additional non-limiting examples of cationic polymers include the polymerized materials such as certain quaternary ammonium salts, copolymers of various materials such as hydroxyethyl cellulose and dialkyldimethyl ammonium chloride, acrylamide and beta methacryloxyethyl trimethyl ammonium methosulfate, the quaternary ammonium salt of methyl and stearyl dimethylaminoethyl methacrylate quaternized with dimethyl sulfate, quaternary ammonium polymer formed by the reaction of diethyl sulfate, a copolymer of vinylpyrrolidone and dimethyl aminoethylmethacrylate, quaternized quars and guar gums and the like.

In embodiments, cationic polymers include: polyquaternium, -5 (the copolymer of acrylamide and beta-methacrylyloxyethyl trimethyl ammonium methosulfate), -6 (a polymer of dimethyl diallyl ammonium chloride), -7 (the polymeric quaternary ammonium salt of acrylamide and dimethyl diallyl ammonium chloride monomers), and combinations of these cationic polymers.

In embodiments, cationic polymers include: hydroxypropyl guar gum, and/or guar hydroxypropyltrimonium chloride.

Quantities of such a cationic polymer are generally a minimum of about 0.05 wt. % of the formulation. Generally, the maximum quantity is no more than about 10 wt. % of the formulation.

In accordance with the present disclosure, cleanser preparatory compositions can be applied to skin in amounts that provide the benefit to the skin of the user, such as in an amount sufficient to remove dirt and oil from the skin. Generally, the cleansers are soap-free and include water, detergent, surfactant, humectants, skin conditioning agent, PH adjustor, extracts, preservatives, fragrance and colorant, however, any cleanser suitable for removing dirt and oil from skin may be used. Suitable cleansers are commercially available and typically include a combination of anionic, cationic, amphoteric and/or non-ionic surfactants in an aqueous vehicle. The cleanser advantageously can include a combination of compounds to compensate for the well known fact that cleansing agents, by their very nature, are not always well tolerated by the skin. The oil-removal feature of a cleanser can result in drying of the skin, and skin irritation. By incorporating various protective agents in the cleanser process the cleanser overcomes shortcomings found in many alternative products. One commercially available preparatory composition is Obagi Nu-Derm® cleanser from OMP, Inc. of Long Beach, CA. The Obagi Nu-Derm® cleanser contains a combination of water, cocamidopropyl betaine, sodium lauroyl oat amino acids, sodium laureth sulfate, glycerin, aloe barbadensis gel, glycerth-7, apricot triethanolamine, sage extract, borage extract, phenoxythanol, methylparaben, propylparaben, ethylparaben, butylparaben, saponins, fragrance, and colorant.

Optionally, foaming gel preparatory composition can be applied in amounts that provide the benefit to the skin of the user, such as in an amount sufficient to remove dirt, oil and/or impurities to clean skin and leave it more receptive to treatment. Generally, foaming gels include water, detergent, surfactant, humectants, skin conditioning agent, PH adjustor, extracts, preservatives, fragrance and colorant; (however any foaming gel may be applied that cleans the skin by removing dirt and/or oil). One commercially available composition is Obagi Nu-Derm® foaming gel from OMP, Inc. of Long Beach, CA. The Obagi Nu-Derm® foaming gel contains a combination of water, sodium lauryl oat amino acids, cocamidopropyl betaine, sodium laureth sulfate, aloe barbadensis gel, alfalfa extract, borage extract, sodium chloride, xantham gum, saponins, phenoxythanol, methylparaben, propylparaben, ethylparaben, butylparaben, fragrance and colorant. This cleanser frees the skin of pollutants without damaging the skin's own natural moisture content.

Optionally, toner can be applied in amounts that provide the benefit to the skin of the user, such as in an amount sufficient to hydrate and tone skin while reducing the pH of the skin. Toner may also help by removing dirt, oils, and grime without overly drying out sensitive skin. Generally, toners include water, skin conditioner, astringent, minerals, moistening agent, vitamins and complexes thereof, anti-microbial, cleanser, extract, surfactant, anti-irritant, fragrance and colorant; however any commercially available skin toner may be used. One commercially available composition is Obagi Nu-Derm® toner available from OMP, Inc. of Long Beach, CA to ameliorate the potentially harsh or drying effects of witch hazel. The Obagi Nu-Derm® toner contains a combination of water, aloe barbadensis gel, witch hazel distillate, potassium alum, sodium PCA, panthenol, DMDM hydantion, polysorbate 80, allantoin, sage extract, calendula officinalis extract, saponins, fragrance, and colorant.

The pre-treatment composition is pre-selected according to the type of drug to be applied to the area to be treated. Other pre-selection factors include the type of skin to be treated, the original presentation of the skin, the type of disease or sickness to be treated, and combinations of these factors.

### DRUGS AND ACTIVE INGREDIENTS

In accordance with the present disclosure, pre-treated skin can also be treated by applying one or more active ingredients to the skin. Suitable topically applied drugs or pharmaceutical ingredients are well known and commercially available. Such compositions include active and inactive ingredients which stabilize and maintain the pharmaceutical or drug composition within the formulation.

Non-limiting examples of drug or active agents which may be applied to the pre-treated skin are listed below. The drugs and active ingredients are applied in amounts that provide a benefit to the skin of a user. While the amount of active used will depend on a number of factors including the specific active chosen and the benefit to be achieved, generally, amounts from about 0.01 to about 10 % by weight of the total active containing composition is suitable. In embodiments, the active is present in an amount from about 0.1 to about 5% by weight of the total composition.

Suitable drugs or active ingredients are categorized in various classes however this classification is not intended to limit the actives in any way to only those actives belonging to the categories herein mentioned.

### Antimicrobial Actives

Antimicrobials which may be applied to a pre-treated area of skin include all antibiotics, antimicrobial agents and antimicrobial peptides. Antibiotics that may be used include *inter alia* dermatologically acceptable salts of tetracycline and tetracycline derivatives, gentamycin, kanamycin, streptomycin, neomycin, capreomycin, lineomycin, paromomycin, tobramycin, erythromycin, triclosan, octopirox, parachlorometa xylenol nystatin, tolnaftate, miconazole hydrochloride, chlorhexidine gluconate, chlorhexidine hydrochloride, methanamine hippurate, methanamine mandelate, minocycline hydrochloride, clindamycin, cleocin, b-lactam derivatives such as aminopenicillin and mixtures thereof. One embodiment for use herein includes chlorhexidine gluconate and triclosan.

Antimicrobial agents that may be used in accordance with the present disclosure either alone or in combination include for example benzoyl peroxide and salicylic acid.

Antimicrobial peptides useful herein are for example magainin, nicin and cecropin.

### Anti-Acne Actives

Anti-acne actives which may be applied to a pre-treated area of skin include all known anti-acne compounds. Anti-acne actives include without limitation keratolytic agents including lactic acid, pyruvic acid, salicylic acids, urea and N-acetylcysteine; retinoids, and retinoid analogs such as tretinoin, cis and trans retinoic acid, retinol and retinol palmitate, isotretinoin-13-cis-retinoic acid; antibiotics and antimicrobial agents such as tetracycline, erythromycin, minocycline, clindamycin, trimethoprim-sulphamethazole and anti-microbial peptides (nicin, for example); steroids, such as hydrocortisone; gamma-linolenic acid and mixtures thereof. Further anti-acne actives that may be used include without limitation benzoyl peroxide; alpha and beta hydroxy acids; sulfacetamide and.sulfur and mixtures thereof. Other actives used herein are salicylic acid, and retinoids.

### Anti-Psoriasis Actives

Anti-psoriasis actives which may be applied to a pre-treated area of skin include all known anti-psoriasis compounds. Anti-psoriasis actives for use in accordance with the present disclosure include without limitation salicylic acid; mometasone furoate; steroids including corticosteroids such as cortisone and oluxclobetasol propionate; 5-fluorouracil; epinephrine; anthralin; vitamin D3 analogs, such as calcipotriene; methotrexate; masprocol; trimethaxate gluconate; retinoids; cyclosporin; paclitaxel; 5-amino levulinic acid; bergasol; tin-ethyl etio purpurin; benzoporphyrin derivatives; antibodies, such as ABX-IL8 antibody, CD11 a monoclonal antibody and ICM3 monoclonal antibody; enzyme inhibitors, including tryptase inhibitor and phospholipase A-2 inhibitors; angiogenesis blocking agents; T-cell blocking agents and mixtures thereof.

### Anti-Eczema Actives

Anti-eczema actives which may be applied to a pre-treated area of skin include all known anti-eczema compounds. Anti-eczema actives useful herein include urea; evening primrose oil; plant extracts; hydrocortisone; an immunomodulator; tar combined with fatty acids obtained from banana; and mixtures thereof.

### Topical Anesthetic Actives

Topical anesthetic actives for use with the present disclosure include tetracaine, lidocaine, editocaine, bupivacaine, pramoxine; and mixtures thereof.

### Anti-inflammatory Actives

Anti-inflammatory actives for use in accordance with the present disclosure include steroidal actives such as hydrocortisone as well as non-steroidal actives including propionic derivatives; acetic acid derivatives; biphenylcarboxylic acid derivatives; fenamic acid derivatives; and oxicams. Examples of anti-inflammatory actives include without limitation acetaminophen, oxaprozin, pranoprofen, benoxaprofen, bucloxic acid, elocon, and mixtures thereof.

### Vitamin Actives

Vitamin actives for use in accordance with the present disclosure include vitamin A and derivatives, including retinoic acid, retinyl aldehyde, retin A, retinyl palmitate, adapalene, and beta-carotene; vitamin B (panthenol, provitamin B5, panthenic acid, vitamin B complex factor); vitamin C (ascorbic acid and salts thereof) and derivatives such as ascorbyl palmitate; vitamin D including calcipotriene (a vitamin D3 analog) vitamin E including its individual constituents alpha-, beta-, gamma-, delta-tocopherol and cotrienols and mixtures thereof and vitamin E derivatives including vitamin E palmitate, vitamin E linolate and vitamin E acetate; vitamin K and derivatives; vitamin Q (ubiquinone) and mixtures thereof. Stable vitamin C formulations are further described below.

### Protein Actives

One class of actives for use in accordance with the present disclosure are proteins and peptides. In principle, any desired protein or peptide may be produced using this technology and oil bodies comprising these recombinant proteins may be incorporated in the emulsions of the present disclosure. Proteins and peptides which may be used in accordance with the present disclosure include enzymes such as proteases (e.g. bromelain, papain, collagenase, elastase), lipases (e.g. phospholipase C), esterases, glucosidases, exfoliating enzymes; antibodies and antibody derived actives, such monoclonal antibodies, polyclonal antibodies, single chain antibodies and the like; reductases; oxidases; peptide hormones; natural structural skin proteins, such as elastin, collagen, reticulin and the like; growth factors such as platelet derived growth factor (PDGF) and epidermis derived growth factor (EGF); anti-oxidants such as superoxide dismutase, catalase and glutathione; free-radical scavenging proteins; DNA-repair enzymes, for example T4 endonuclease 5 and P53; antimicrobial peptides, such as magainin and cecropin; a milk protein; a silk protein or peptide; and any active fragments, derivatives of these proteins and peptides; and mixtures thereof.

### Anti-wrinkle and anti-aging actives

Anti-wrinkle and anti-aging actives for use in accordance with the present disclosure include without limitation hydroxy acids including C₂ -C₃₀ alpha-hydroxy acids such as glycolic acid, lactic acid, 2-hydroxy butanoic acid, malic acid, citric acid tartaric acid, alpha-hydroxyethanoic acid, hydroxycaprylic acid and the like; beta hydroxy acids including salicylic acid and polyhydroxy acids including gluconolactone (G4); and mixtures of these acids. Further anti-wrinkle agents include retinoic acid, gamma-linolenic acid; fruit acids, sugar cane extract and glycomer in cross-linked alpha nutrium; and mixtures thereof. Skin peel agents for example phenol, phytic acid and acetic acid may also be used in accordance with the present disclosure. Salicylic acid, lactic acid and glycolic acid are also for use herein.

### Whitening and Bleaching Actives

Whitening and bleaching agents include kojic acid, lactic acid, ascorbyl acid and derivatives such as magnesium ascorbyl phosphate; arbutin; hydroquinone, and licorice root.

### Sunless Tanning Actives

Sunless tanning actives include dihydroxyacetone (DHA); glyceryl aldehyde; tyrosine and tyrosine derivatives such as malyltyrosine, tyrosine glucosinate, and ethyl tyrosine; phospho-DOPA, indoles and derivatives; and mixtures thereof.

### Non-Steroidal Drugs

Other useful drugs include non-steroid drugs for releasing pain and include, for example, ketoprofen, fellinac, ibuprofen, pirorican, bis (indole) alkaloid compounds and the like.

### Miscellaneous Active Ingredients

Further active ingredients for use in accordance with the present disclosure include an amino acid and amino acid derivative; an insect repellant; a fungicide (such as ketoconazole, itraconazole, saperconazole); an anti-viral agent (such as acyclovir); an anti-cancer agent; a plant extract; an anti-hemorrhoid compound; an anti-dandruff compound; a hair-growth stimulating compound such as minoxodil and Revivogen; a hair loss stimulating compound; a nucleic acid (DNA, RNA and derivatives); an anti-scabies agent (such as permethrin); an anti-wart agent (such as podophyllotoxin); and mixtures thereof.

Thus compositions for use in accordance with the present disclosure include bacitracin, cortisone-containing compositions; antibiotics, such as Neosporin; hydroquinone; antibacterial compositions; gastrointestinal drugs; anti-fungal drugs; addiction modifiers; cardio-vascular, cardio-pulmonary and pulmonary treatment drugs; anti-inflammatory compounds; hypo-allergenic compounds, analgesic compounds, hypertension treatment drugs; diabetes treatment drugs or pharmaceutical compounds, neurological treatments and the like, as well as sequential applications thereof, and the like. Antibiotics such as nicotinamide, clindamycin and erythromycin can be topically applied in treating acne. Other useful antibiotics and drugs include, for example, 5F-U for treating certain types of cancer, minoxidil, and the like.

### Stable Vitamin C Compositions

The active containing compositions of the present disclosure includes stable vitamin C compositions formulated in a manner which enables the vitamin C to remain stable when mixed with water. Compositions in accordance with some embodiments of this disclosure are effective in enhancing the penetration of vitamin C in the skin.

The compositions of the present disclosure contain vitamin C and a unique mixture of ingredients in an aqueous solution. The term "vitamin C" as used herein applies to substances that possess antiscorbutic activity. Such substances include, for example, L-ascorbic acid, commonly called ascorbic acid, salts of L-ascorbic acid, L-dehydroascorbic acid and salts of L-dehydroasorbic acid. L-ascorbic acid is a well known compound of general formula:

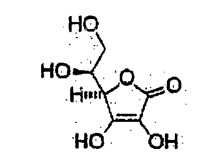

Suitable salt forms of vitamin C include any salt formed from the neutralization of ascorbic acid. Illustrative examples include sodium ascorbate formed by the neutralization ascorbic acid with sodium to form L-ascorbic acid-monosodium salt. Other useful forms include calcium ascorbate, magnesium ascorbate, potassium ascorbate, manganese ascorbate, zinc ascorbate, molybdenum ascorbate, chromium ascorbate, and combinations thereof.

The vitamin C is present in amounts that provide a benefit to the skin of a user. In embodiments, vitamin C is present in an amount sufficient to promote therapeutic or corrective treatment of a user's skin. The vitamin C present may be in acidic form, salt form, or mixtures thereof. As an illustrative example, amounts of vitamin C between about 5 and about 40% by weight of the total composition may be suitable. In embodiments, vitamin C is present in an amount between about 15 and about 25% by weight of the total composition. In other embodiments, the amount of vitamin C is between about 18 and about 22% by weight of the total composition.

The aqueous solution of the stable Vitamin C composition can further include water, one or more reducing sugars, one or more antimicrobial preservatives, one or more salts, one or more reducing agents; one or more surfactants, one or more conditioners such as Na Hyalurate, fragrance, and combinations thereof. In embodiments, purified water is used, such as, for example de-ionized water or USP water.

Suitable reducing sugars for stable vitamin C compositions include sugars with a ketone or aldehyde group such that the sugar is capable of acting as a reducing agent. Illustrative examples of reducing sugars include mannitol, sorbitol, xylitol, maltitol, lactitol, or combinations thereof. In vitamin C and reducing sugar compositions, it is believed that the reducing sugar oxidizes first and delays the start of any oxidation of the vitamin C, so that excessive oxidation in water is delayed or totally avoided. In embodiments, the reducing sugars are present between about 0.1 % w/v to about 10.0% w/v of the formulation. In embodiments, the reducing sugars are present between about 0.5% w/v to about 5.0% w/v of the formulation.

Optionally, the reducing sugars can be mixed with water to form a reducing sugar solution that can be used to formulate a stable vitamin C composition in accordance with this disclosure. The reducing sugar solution may contain, for example, reducing sugar in an amount between about 1% to about 99% by weight of the total composition. In embodiments, the reducing sugar solution may contain about 70% by weight of the total composition. The amount of reducing sugar solution used to formulate the stable vitamin C composition will depend upon a number of facts including the concentration of reducing sugar in the solution. Typically, however, for a 70% solution, the reducing sugar solution may be added to the composition to between about 0.25% v/v to about 10.0% v/v of the formulation. In embodiments, such reducing sugar solution is admixed between about 1 % to about 5% v/v of the total formulation.

In vitamin C composition embodiments, sorbitol can be used as a reducing sugar. Sorbitol, also known as glucitol is a sugar alcohol having the general formula:

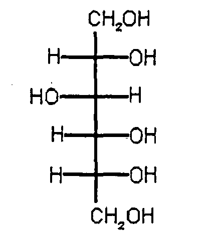

Sorbitol is a sugar alcohol (also known as polyol, polyhydric alcohol, or polyalcohol) which is a hydrogenated form of carbohydrate, whose carbonyl group (aldehyde or ketone, reducing sugar) has been reduced to a primary or secondary hydroxyl group. In one embodiment, sorbitol is mixed with water to form a 70% solution suitable for use as an ingredient in forming compositions in accordance with the present disclosure.

Antimicrobial preservatives can be used to prevent or inhibit the growth of microorganism which could present a risk of infection to the user or degrade the vitamin C. Thus, suitable antimicrobial preservatives include ingredients capable of retarding the oxidation of vitamin C and/or extending the shelf-life of active ingredients. The properties of these antimicrobial substances typically include chemical groups that are aggressive towards living cells. Examples of suitable preservatives include quaternary ammonium salts, phenoxyethanol, amine salts, Na Metabisulfite and combinations thereof. The antimicrobial preservatives may be present between about 0.1 % w/w to about 5.0% w/w of the formulation.

Suitable salts that may be employed in making stable vitamin C compositions in accordance with the present disclosure include acid and base addition salts. Illustrative examples of such acid salts include: inorganic acid addition salts such as hydrochloride, sulfate, and phosphate; and organic acid addition salts such as acetate, maleate, fumarate, tartrate, and citrate. Examples of suitable basic salts include: metal salts such as the alkali metal salts such as the sodium salt and potassium salt; alkaline earth metal salts such as magnesium salt and calcium salt; and other salts such as aluminum salt, and zinc salt. Examples of suitable ammonium salts are ammonium salt and tetramethylammonium salt. Examples of suitable amine addition salts are salts with morpholine and piperidine. Examples of suitable amino acid addition salts include salts with lysine, glycine, and phenylalanine. The one or more salts may be present in a between about 0.01 % w/w to about 4.0% w/w of the formulation. In embodiments mixtures of salts have been found to further promote stability, especially when combined with a reducing sugar such as sorbitol. In one embodiment, Ca hydroxide (e.g., between about 0.01 - 0.5% w/v of the total formulation) is combined with Zn Chloride (e.g., between about 0.01 - 2.0% w/v of the total formulation). The combination of salt admixtures with reducing sugar was found to promote stability of aqueous ascorbyl acid solutions.

In embodiments, alkaline earth metal salts such as magnesium and calcium salts may be provided in a unique stability promoting admixture. It is believed that the combination of such metal ions in solution promotes the stability of the compositions. Other salt mixtures such as zinc salts and aluminum salts also promote stability.

In embodiments, the stable Vitamin C composition can optionally include surfactants. Suitable surfactants for use with the compositions of the present disclosure include ionic or nonionic surfactants, used alone or in admixture. Illustrative surfactants include cetearyl alcohol and sodium cetearyl sulfate, alkyldimethylbenzylamines, PEG-1000 monocetyl ether, or quaternary ammonium salts such as alkyl trimethyl ammonium bromide; polyol ester glycerol monostearate and potassium stearate, sodium lauryl sulfate, ethoxylated fatty alcohols and combinations thereof. Fatty acids like stearic acids may be included to regulate the consistency of the composition. Optionally, polymers such as carbomers can be included in the present composition. Particularly useful surfactants for use in the aqueous phase are sodium lauryl sulfate, saponins or combinations thereof. The surfactants may be present between about 0.0 and about 20% by weight of the total composition. In embodiments, the surfactants are present in an amount between about 0.1 and about 5% by weight of the total composition.

The inclusion of various surfactants in compositions of the present disclosure increases the percutaneous absorption of vitamin C when solutions are applied to skin. It is believed that by adding surfactants to the stable ascorbic acid solutions, the surface tension of the solution is decreased allowing for better absorption through skin. Thus methods of applying surfactant containing solutions to skin in order to increase the levels of vitamin C absorbed into the skin are also described herein. Accordingly, surfactant may also be included in the compositions of the present disclosure in amounts sufficient to increase the absorption of vitamin C through skin. For example, by adding SLS (30% solution) in an amount of about 0.1 to 5% by weight of the total composition, and then applying the solution to skin, the percutaneous absorption of vitamin C through skin is increased in comparison to vitamin C solutions that do not have surfactant.

The pH of the aqueous solution can be adjusted to be between about 2 to about 6, and, in some particularly useful embodiments below 5. The pH of the composition ensures that most of the ascorbic acid remains in the protonated, uncharged form. The protonated form of ascorbic acid used in compositions of the present disclosure is believed to remove the ionic repulsion of the two oxygen groups, thus helping to stabilize the molecule. Also because the protonated form of ascorbic acid is uncharged, entry into the skin (which itself has a pH of about 3-5) is believed to be facilitated. Agents suitable for adjusting the pH of the aqueous phase include, but are not limited to citric acid, phosphoric acid, lactic acid or glycolic acid. The pH adjustment agents may be present between about 0.01 to about 5% by weight of the total composition. In embodiments, the pH adjustment agent is present in an amount of about 0.1 to about 1.0% by weight of the total composition.

Suitable reducing agents that can be used in the stable Vitamin C compositions include, but are not limited to propyl gallate and sulfites, including but not limited to sulfites, bisulfites, metabisulfites, their salts, and their derivatives, in embodiments sodium metabisulfite. Since vitamin C has a tendency to oxidize, these antioxidants may be advantageous because they have greater tendencies to oxidize than vitamin C. Sodium metabisulfite has the added advantage that it does not discolor by oxidation. In vitamin C and sodium metabisulfite compositions, it is believed that the sodium metabisulfite oxidizes first and delays the start of any oxidation of the vitamin C, so that excessive oxidation is delayed or totally avoided. The reducing agents may be present between about 0.1 and about 10% by weight of the total composition. In some embodiments, the reducing agents are present in an amount of about 0.5 to about 5% by weight of the total composition.

The aqueous phase can be prepared by mixing the various ingredients while mixing and heating to 70-75°C.

Other suitable optional ingredients include moisturizing agents such as Na Hyalurate solution and fragrance. Na Hyalurate moisturizing agent that is synonymous with and refers to hyaluronic acid, sodium salt; sodium hyaluronate; hyaluronic acid; or sodium hyalurate and has the general formula (C₁₄H₂₀NO₁₁Na)ₙ. Na hyalurate 1% solution may be present, for example, in an amount of about 0.001 to about 0.2% w/v of the total composition, or in amounts the effectively moisturize the formulations.

The viscosity of the final vitamin C composition can be between about 30 to 10,000 centipoise (cps), in embodiments between about 30 and about 250 cps. The specific gravity of the final Vitamin C composition can be between about 1 and 115 cps, in embodiments between about 102 and about 106 cps.

The final vitamin C composition may be a substantially clear, viscous liquid to a semi-viscous lotion. Those skilled in the art will envision testing to confirm the shelf life of the products described herein. Further testing methodology is described below.

The present disclosure also relates to methods of pre-treating skin to increase the percutaneous absorption of active agents such as Vitamin C there through. It has been found that the application of cationic constituent such as cationic salt or cation containing solution to skin prior to the application of drug or active agent compositions suitable for topical application to skin increases the percutaneous absorption of drug or active agent into the skin. Cationic constituents or solutions thereof are applied to skin in amounts sufficient to increase the percutaneous absorption of vitamin C. For example, a small volume of a cationic salt-containing pretreatment solution (such as, for example, an aqueous 5% alkyldimethylbenzylamine solution) may be applied to skin prior to the application of a drug or active agent. The skin is dried and the drug or active agent is added to the skin.

In a particularly useful method in accordance with this disclosure, a cleanser is used to cleanse the area of skin to be treated and the residue wiped off. Suitable cleanser includes the Obagi Nu-Derm Cleanser and Obagi Nu-Derm foaming gel both commercially available from OMP, Inc. of Long Beach, CA. Cleanser is applied in an amount suitable for cleaning the skin area to be cleaned. Subsequently toner may be applied to the cleansed area and allowed to dry. Suitable toner includes Obagi Nu-Derm Toner commercially available from OMP, Inc. of Long Beach, CA. Toner is applied in an amount suitable for toning the skin area to be toned. In embodiments, a cationic salt formulation is applied in amounts suitable for coating the cleansed, toned skin with positively charged molecules. An active ingredient containing composition such as Vitamin C is prepared and then applied to the skin in an amount suitable to obtain the desired effect. The steps of cleansing, toning, and/or charging the skin increase the percutaneous absorption of active drug ingredient through the skin.

The following examples further illustrate this disclosure.

### EXAMPLES

Example 1 below shows suitable stable Vitamin C active ingredient compositions in accordance with the present disclosure.

| **Ingredient** | **% of solution (w/v)** |
|---|---|
| Water | 60-96 |
| Sorbitol 70% | 0.1-10 |
| Ca Hydroxide | 0.10-0.5 |
| Zn Chloride | 0.10-2 |
| Na Hyalurate | 0.001-0.02 |
| Ascorbic acid | 5-40 |
| SLS (30% solution) | 0-5 |
| Phenoxyethanol | 0.1-1 |
| Fragrance | 0.0-5 |
| Alkydimethylbenzylamine | 0-5 |

Example 2 below shows other suitable active ingredient containing compositions in accordance with the present disclosure.

| **Ingredient** | **% of solution (w/v)** |
|---|---|
| Water | 60-96 |
| Reducing Sugar | 0.1-10 |
| Metal Salt or mixtures thereof | 0.10-5 |
| Moisturizing Agent | 0.0-0.02 |
| Ascorbic acid | 5-40 |
| Antimicrobial | 0.0-1 |
| Surfactant | 0.0-5 |
| Fragrance | 0.0-5 |

The compositions of the present disclosure may be packaged in suitable containers such as tubes or bottles. Suitable containers are commercially available from a variety of suppliers. A wide variety of containers and suppliers are listed in the CPC Packaging Directory. (See, Buyers' Guide under "Containers" at www.cpcpkg.com). In embodiments, containers are selected with low oxygen permeability. Suitable containers include high density polyethylene and the like.

### Example 3

### Stability study of stable Vitamin C active containing composition

In vitamin C compositions without reducing sugar, an aqueous solution of 5% ascorbic acid will likely decompose to less than 90% of the concentration at room temperature in 4 weeks time.

Conversely, the stability of compositions made in accordance with the present disclosure show improved stability. Such compositions were evaluated by placing aliquots of each example in an oven at 5, 25, 30 and 40 degrees Centigrade for predetermined time periods and at the end of each time period analyzing the amount of vitamin C present in the composition.

The following results were observed with compositions in accordance with example 1 having 20% initial vitamin C concentration, sorbitol 70%, Ca hydroxide, Zn chloride, Na hyaluronate 1%, SLS (30% solution), phenoxyethanol and fragrance.

| **Stability of formulation of example No. 1** | **% Vitamin C** |
|---|---|
| Initial amount of Vit. C | 20% |
| 6 weeks at 40C: | 16.52% |
| 3 months at 25C: | 17.50% |
| 3 months at 40C: | 13.23% |
| 8 months at 25C | 16% |

Conversely, vitamin C composition without reducing sugar showed only 9.7% vitamin C remaining after 3 months at 40°C.

### Example 4

### Percutaneous Absorption study:

The in-vitro percutaneous absorption of vitamin C formulations were compared using intact human cadaver skin. Cumulative transdermal absorption of radiolabeled [¹⁴C] L-ascorbic acid was measured at 24 hours. The human cadaver skin was obtained from a single donor and dermatomed to approximately 500 micron thickness. The skin samples were mounted on Franz static diffusion glass chambers. The skin surfaces of approximately 1.77 cm² were washed with .5 ml of water at 37°C for 10 seconds. The water was aspirated and the surface pad dried. The following treatments were performed.
Treatment A. 15 mg of a formulation in accordance with example 1 having 25% ascorbic acid, reducing sugar, and metal ions was applied to 1.77 cm² of human cadaver skin samples.
Treatment B: Skin pretreated with 15 mg of pretreatment solution (5% benzalkonium chloride, a cationic salt). The skin was dried, and then 15 mg of formulation of Treatment A was applied to the skin.

**TABLE A**

| Treatment | AMOUNT OF ASCORBIC ACID ABSORBED DERMIS (24 hrs.) | |
|---|---|---|
| | In micrograms | as % of amount applied |
| A | 26.5 | 8.0 |
| B | 93.1 | 33.6 |

Pre-treating the skin by topically applying a cationic salt or solution thereof to the skin enhanced absorption of vitamin C into the dermis, compared to skin that was not pretreated. Dermal levels were observed as increasing by approximately 4 times.

### Example 5

### Percutaneous Absorption study:

The in-vitro percutaneous absorption of vitamin C formulations are compared using intact human cadaver skin. Cumulative transdermal absorption of radiolabeled [¹⁴C] L-ascorbic acid is measured. The human cadaver skin is obtained from a single donor and dermatomed to approximately 500 micron thickness. The skin samples are mounted on Franz static diffusion glass chambers. The skin surfaces of approximately 1.77 cm² arewashed with .5 ml of water at 37°C for 10 seconds. The water is aspirated and the surface pad dried. The following treatments are performed.
Treatment A. 15 mg of a formulation in accordance with example 1 having 25% ascorbic acid, reducing sugar, and metal ions is applied to 1.77 cm² of human cadaver skin samples.
Treatment B: Skin pretreated with 15 mg of pretreatment solution (5% polyquaternium, -5 (the copolymer of acrylamide and beta-methacrylyloxyethyl trimethyl ammonium methosulfate) (a cationic polymer). The skin is dried, and then 15 mg of formulation of Treatment A is applied to the skin.

Percutaneous absorption of Vitamin C in Treatment B is expected to be greater than that of Treatment A.

### Example 6

### Percutaneous Absorption study:

The in-vitro percutaneous absorption of vitamin C formulations are compared using intact human cadaver skin. Cumulative transdermal absorption of radiolabeled [¹⁴C] L-ascorbic acid is measured. The human cadaver skin is obtained from a single donor and dermatomed to approximately 500 micron thickness. The skin samples are mounted on Franz static diffusion glass chambers. The skin surfaces of approximately 1.77 cm² are washed with .5 ml of water at 37°C for 10 seconds. The water is aspirated and the surface pad dried. The following treatments are performed.
Treatment A. 15 mg of a formulation in accordance with example 1 having 25% ascorbic acid, reducing sugar, and metal ions is applied to 1.77 cm² of human cadaver skin samples.
Treatment B: Skin is pretreated by cleansing with cleanser composition and toning with a toner composition. Skin is further pretreated with 15 mg of pretreatment solution (5% arginine, a basic amino acid). The skin is dried, and then 15 mg of formulation of Treatment A is applied to the skin.

Percutaneous absorption of Vitamin C in Treatment B is expected to be greater than that of Treatment A.

### Example 7

### Percutaneous Absorption study:

The in-vitro percutaneous absorption of vitamin C formulations are compared using intact human cadaver skin. Cumulative transdermal absorption of radiolabeled [¹⁴C] L-ascorbic acid is measured. The human cadaver skin is obtained from a single donor and dermatomed to approximately 500 micron thickness. The skin samples are mounted on Franz static diffusion glass chambers. The skin surfaces of approximately 1.77 cm² arewashed with .5 ml of water at 37°C for 10 seconds. The water is aspirated and the surface pad dried. The following treatments are performed.
Treatment A. 15 mg of a formulation in accordance with example 1 having 25% ascorbic acid, reducing sugar, and metal ions is applied to 1.77 cm² of human cadaver skin samples.
Treatment B: Skin pretreated with cleanser, allowed to dry, then 15 mg of pretreatment solution (5% Guar hydroxypropyltrimonium chloride) (a cationic polymer). The skin is dried, and then 15 mg of formulation of Treatment A is applied to the skin.

Percutaneous absorption of Vitamin C in Treatment B is expected to be greater than that of Treatment A.

### Example 8

### Percutaneous Absorption study:

The in-vitro percutaneous absorption of tetracycline (topical) formulation is compared using intact human cadaver skin. Cumulative transdermal absorption of labeled tetracycline is measured. The human cadaver skin is obtained from a single donor and dermatomed to approximately 500 micron thickness. The skin samples are mounted on Franz static diffusion glass chambers. The skin surfaces of approximately 1.77 cm² are washed with 0.5 ml of water at 37°C for 10 seconds. The water is aspirated and the surface pad dried. The following treatments are performed. Treatment A. 5 ml of a topical tetracycline formulation is applied to 1.77 cm² of human cadaver skin samples.

Treatment B: Skin pretreated with 15 ml of pretreatment solution (5% arginine, a basic amino acid). The skin is dried, and then 5 ml of formulation of Treatment A is applied to the skin.

Percutaneous absorption of Vitamin C in Treatment B is expected to be greater than that of Treatment A.

While several embodiments of the disclosure have been described, it is not intended that the disclosure be limited thereto, as it is intended that the disclosure be as broad in scope as the art will allow and that the specification be read likewise.

## Claims

1. Use of a salt of an alkyldimethylbenzylamine for the manufacture of a composition for improving the percutaneous absorption of ascorbic acid topically applied to the skin of a user, comprising:
pretreating an area of a user's skin by topically applying a salt of an alkyldimethylbenzylamine; and
topically applying a composition containing ascorbic acid to the pretreated area.

2. Use according to claim 1, wherein the salt of an alkyldimethylbenzylamine is a benzalkonium chloride.

3. Use according to one of the preceding claims, wherein the step of pre-treating the surface comprises applying a preparatory composition to the surface, wherein the preparatory composition comprises cleaner, foaming gel, toner or combinations thereof.

## Patentansprüche

1. Verwendung eines Salzes eines Alkyldimethylbenzylamins für die Herstellung einer Zusammensetzung zum Verbessern der perkutanen Absorption von Ascorbinsäure, die topisch auf die Haut eines Anwenders aufgebracht wird, umfassend:
Vorbehandeln einer Hautfläche eines Anwenders durch topisches Auftragen eines Salzes eines Alkyldimethylbenzylamins; und
topisches Auftragen einer Ascorbinsäure enthaltenden Zusammensetzung auf die vorbehandelte Fläche.

2. Verwendung nach Anspruch 1, wobei das Salz eines Alkyldimethylbenzylamins ein Benzalkoniumchlorid ist.

3. Verwendung nach einem der vorhergehenden Ansprüche, wobei der Schritt des Vorbehandelns der Oberfläche das Auftragen einer vorbereitenden Zusammensetzung auf die Oberfläche umfasst, wobei die vorbereitende Zusammensetzung Reinigungsmittel, schäumendes Gel, Gesichtswasser oder Kombinationen derselben umfasst.

## Revendications

1. Utilisation d'un sel d'une alkyldiméthylbenzylamine pour la fabrication d'une composition pour améliorer l'absorption percutanée d'acide ascorbique appliqué topiquement sur la peau d'un utilisateur, comprenant :
le prétraitement d'une zone de peau d'un utilisateur en appliquant topiquement un sel d'une alkyldiméthylbenzylamine ; et
l'application topique d'une composition contenant de l'acide ascorbique sur la zone prétraitée.

2. Utilisation selon la revendication 1, dans laquelle le sel d'une alkyldiméthylbenzylamine est un chlorure de benzalkonium.

3. Utilisation selon l'une des revendications précédentes, dans laquelle l'étape de prétraitement de la surface comprend l'application d'une composition préparatoire sur la surface, dans laquelle la composition préparatoire comprend un nettoyant, du gel moussant, un tonique ou des combinaisons de ceux-ci.
